(19) Europäisches Patentamt
European Patent Office
Office européen des brevets

(11) **EP 2 212 820 B1**

(12) **FASCICULE DE BREVET EUROPEEN**

(45) Date de publication et mention de la délivrance du brevet:
**04.01.2012 Bulletin 2012/01**

(21) Numéro de dépôt: **08871115.5**

(22) Date de dépôt: **23.10.2008**

(51) Int Cl.:
***G06F 19/10*** ^(2011.01)

(86) Numéro de dépôt international:
**PCT/FR2008/001488**

(87) Numéro de publication internationale:
**WO 2009/090344 (23.07.2009 Gazette 2009/30)**

(54) **MESURE D'UNE POPULATION D'ACIDES NUCLÉIQUES, EN PARTICULIER PAR PCR EN TEMPS RÉEL**

ECHTZEITMESSUNG EINER POPULATION VON NUKLEINSÄUREN, INSBESONDERE DURCH PCR

REAL-TIME MEASURE OF A POPULATION OF NUCLEIC ACIDS, IN PARTICULAR BY PCR

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **25.10.2007 FR 0707508**

(43) Date de publication de la demande:
**04.08.2010 Bulletin 2010/31**

(73) Titulaire: **Bio-Rad Innovations**
**92430 Marnes-La-Coquette (FR)**

(72) Inventeurs:
• **JAHAN, Virginie**
  **F-92500 Rueil Malmaison (FR)**
• **KAMINSKI, Karine**
  **F-34990 Juvignac (FR)**

(74) Mandataire: **Plaçais, Jean Yves**
**Cabinet Netter**
**36, avenue Hoche**
**75008 Paris (FR)**

(56) Documents cités:
**WO-A-2006/048337**

• **RAMAKERS C ET AL: "Assumption-free analysis of quantitative real-time polymerase chain reaction (PCR) data" NEUROSCIENCE LETTERS, LIMERICK, IE, vol. 339, no. 1, 13 mars 2003 (2003-03-13), pages 62-66, XP002330743 ISSN: 0304-3940**
• **LIU W ET AL: "VALIDATION OF A QUANTITATIVE METHOD FOR REAL TIME PCR KINETICS" BIOCHEMICAL AND BIOPHYSICAL RESEARCH COMMUNICATIONS, ACADEMIC PRESS INC. ORLANDO, FL, US, vol. 294, no. 2, 7 juin 2002 (2002-06-07), pages 347-353, XP002319698 ISSN: 0006-291X**
• **SWILLENS S ET AL: "Instant evaluation of the absolute initial number of cDNA copies from a single real-time PCR curve" NUCLEIC ACIDS RESEARCH, OXFORD UNIVERSITY PRESS, SURREY, GB, vol. 32, no. 6, 1 janvier 2004 (2004-01-01), pages E56.1-E56.6, XP002330744 ISSN: 0305-1048**
• **PFAFFL M W: "A new mathematical model for relative quantification in real-time RT-PCR" NUCLEIC ACIDS RESEARCH, OXFORD UNIVERSITY PRESS, SURREY, GB, vol. 29, no. 9, 1 mai 2001 (2001-05-01), pages E45.2002-E45.2007, XP002330745 ISSN: 0305-1048**

EP 2 212 820 B1

**Description**

La présente invention concerne un procédé de mesure d'une quantité initiale d'acides nucléiques contenus dans un échantillon d'intérêt et soumise à une amplification. L'invention trouve notamment une application avantageuse dans le suivi thérapeutique des maladies infectieuses, mais aussi dans le domaine de la génétique et de la cancérologie, et d'une façon plus générale, quelque soit le domaine d'application comme par exemple le domaine vétérinaire, l'agro-alimentaire, le domaine végétal.

Le domaine concerné est celui des techniques d'amplification génique permettant de déterminer une quantité initiale d'une séquence d'acides nucléiques d'intérêt présente dans un échantillon et notamment les techniques d'amplification permettant la quantification en temps réel, comme la technique de PCR (Réaction en chaîne par polymérase, en anglais "Polymerase Chain Reaction") en temps réel, par exemple.

La présente invention trouve une application avantageuse mais non limitative à la détermination d'une quantité initiale d'acides nucléiques dans un échantillon soumis à une réaction de PCR en temps réel pour évaluer le nombre de copies d'acides nucléiques d'agents infectieux (virus, bactéries, levures, etc.) présent dans un échantillon biologique (sérum, plasma, sang, expectorations, etc..). Cette technique peut travailler sur l'ADN ou l'ARN. On se référera ici généralement et sauf mention contraire à l'ADN, sans pour autant se restreindre à cet acide nucléique.

La PCR fait intervenir des variations de température permettant la dissociation de l'ADN en deux brins ("dénaturation"), puis l'hybridation d'une amorce sur chaque brin de l'ADN ainsi dénaturé. Chaque amorce est donc spécifique, et complémentaire d'un des deux brins d'ADN. L'action d'une ADN polymérase conduit ensuite à la synthèse de nouveaux brins complémentaires de chaque brin initialement utilisé comme matrice. Cela forme une suite d'étapes "dénaturation/hybridation/synthèse de brins", qui est répétée cycliquement.

La PCR en temps réel est une technique permettant l'amplification et la détection simultanée d'une ou plusieurs séquences différentes d'acides nucléiques cibles. La détection peut se faire au cours du temps et peut être quantitative.

La technique de PCR quantitative en temps réel fait intervenir un ou plusieurs marqueurs d'ADN, par exemple fluorescents, permettant la quantification de la séquence nucléique (ou des séquences nucléiques) à détecter. Cette technique utilise généralement la comparaison de l'échantillon à analyser avec un standard, ou avec une gamme de standards. Un standard est un échantillon contenant une quantité connue d'acides nucléiques. Il subit en principe les mêmes traitements que l'échantillon à analyser. Pour l'échantillon d'intérêt comme pour le standard, la fluorescence évolue proportionnellement à la quantité d'acides nucléiques présents dans l'échantillon avant et pendant la réaction. Ceci sera détaillé plus loin dans la suite de la description.

Par ailleurs, la PCR quantitative en temps réel se déroule en plusieurs phases, dont les dynamiques réactionnelles sont différentes, comme on le verra plus loin. Par conséquent, on utilise généralement plusieurs points de mesure, afin de réaliser une poursuite de la dynamique réactionnelle.

Tout ceci fait que les techniques de PCR temps réel pour la quantification d'un échantillon d'intérêt, sont lourdes à mettre en oeuvre, ce qui nuit à la généralité de leur utilisation, partout où leur intérêt est manifeste.

La présente invention vient améliorer la situation.

A cet effet, l'invention vient introduire un procédé de mesure du taux d'acides nucléiques d'une séquence cible dans un échantillon d'intérêt, comprenant les étapes de :

a. soumettre l'échantillon d'intérêt à un traitement d'amplification, avec un lot de réactifs comprenant au moins un marqueur d'acide(s) nucléique(s) spécifique à ladite séquence cible, ledit traitement d'amplification comportant des cycles d'amplification successifs (i ; de 1 à n),
b. mesurer une grandeur physique ($F_i$) représentative de l'évolution du marqueur pour une partie au moins des cycles d'amplification,
c. exprimer un paramètre ($F_0$) représentatif de la grandeur physique du marqueur d'acide(s) nucléique(s) avant tout cycle d'amplification, en utilisant les mesures effectuées à l'étape b.,
d. estimer l'effectif initial ($N_0^{éch.}$) en acide(s) nucléique(s) de la séquence cible à l'aide d'une loi de conversion, comportant des paramètres contextuels, et appliquée audit paramètre ($F_0$) exprimé à l'étape c.,

À l'étape d., lesdits paramètres contextuels sont des paramètres de référence préenregistrés, ces paramètres étant sensiblement indépendants d'une partie au moins des conditions expérimentales.

Les paramètres de référence mentionnés ci-dessus sont déterminés à l'avance à partir du comportement d'au moins un échantillon de référence de même format biologique que l'échantillon d'intérêt et d'effectif initial $N_0^{réf}$ connu en acide(s) nucléique(s).

Selon un mode de réalisation, la loi de conversion à l'étape d., peut être appliquée directement, sans nécessiter de correction par l'exécution en parallèle des étapes a., b. et c. pour au moins un calibrateur.

Ce mode de réalisation simplifie donc la mise oeuvre du procédé pour l'opérateur final en réduisant les temps opératoires et conduit aussi à une réduction des coûts de réactifs non négligeable, notamment dans le domaine de la santé publique. De plus, grâce aux paramètres de référence préenregistrés, on évite une manipulation d'éléments qui classiquement accompagnent une réaction d'amplification. Notamment on évite l'utilisation d'échantillons standards ce qui permet d'augmenter le nombre de puits disponibles pour tester les échantillons à analyser (gain de place).

Un autre mode de réalisation prévoit que l'étape d. comprend une correction de l'effectif initial ($N_0^{éch}$) estimé. Cette correction est fondée sur une exécution à l'avance des étapes a., b. et c. pour au moins un calibrateur (EQC) d'effectif initial en acide(s) nucléique(s) et de paramètre ($F_0^{EQC}$) représentatif de la grandeur physique du marqueur d'acide(s) nucléique(s) avant tout cycle d'amplification connus

Cette correction de l'effectif initial ($N_0^{éch}$) estimé peut être réalisée par les étapes suivantes :

- établir une loi correctrice entre :

  • ledit paramètre ($F_0^{EQC}$) représentatif de la grandeur physique du marqueur d'acide(s) nucléique(s) avant tout cycle d'amplification connu d'une part et,
  • le paramètre ($F_0^{EQCmesuré}$) représentatif de la grandeur physique du marqueur d'acide(s) nucléique(s) avant tout cycle d'amplification effectivement exprimé du calibrateur,

- appliquer ladite loi correctrice à ladite loi de conversion comportant les paramètres contextuels de l'étape d., pour l'estimation dudit effectif initial ($N_0^{éch.}$) en acide(s) nucléique(s) de la séquence cible, présente dans l'échantillon d'intérêt.

Pour la correction sus-mentionnée, le calibrateur (EQC) d'effectif initial connu se fonde sur une substance biologique propre à former un contrôle positif pour l'échantillon d'intérêt.

Dans ce mode de réalisation, la correction ne génère donc pas l'ajout de tubes réactionnels supplémentaires, ce qui est avantageux par rapport aux techniques de quantification connues de l'art antérieur.

À l'étape c. du procédé l'expression du paramètre ($F_0$) fait intervenir, au moins :

- un paramètre relatif au basculement entre une première phase de rendement d'amplification constant et une seconde phase de rendement d'amplification non-constant,
- un paramètre relatif au rendement d'amplification constant lors de ladite première phase, et
- un paramètre relatif au rendement d'amplification non-constant lors de ladite seconde phase.

Le marqueur d'acides nucléiques peut être un marqueur fluorescent.

L'échantillon d'intérêt peut être un prélèvement biologique susceptible de comprendre un agent pathogène.

La réaction d'amplification utilisée peut être une Réaction en Chaîne par Polymérase (PCR) en temps réel.

Les paramètres de référence présentent préférentiellement l'avantage d'être indépendants d'au moins un des éléments du groupe de conditions expérimentales suivantes : appareil utilisé, type d'appareil utilisé, opérateur, période de validité du lot de réactifs, mode d'extraction des acides nucléiques de la séquence cible.

Selon un autre mode de réalisation et dans le cadre d'un traitement d'amplification dite PCR multiplex, l'échantillon d'intérêt peut comprendre plusieurs séquences cibles distinctes à quantifier.

La présente invention vise également un appareil pour mesurer le taux d'acides nucléiques d'au moins une séquence cible dans un échantillon d'intérêt, comprenant :

  a. un élément de support pour supporter au moins un échantillon comprenant un lot de réactifs et la séquence cible avec au moins un marqueur d'acide(s) nucléique(s) spécifique à ladite séquence cible,
  b. une unité d'amplification pour soumettre l'échantillon d'intérêt à un traitement d'amplification comportant des cycles d'amplification successifs (i ; de 1 à n),
  c. une unité de mesure pour mesurer une grandeur physique ($F_i$) représentative de l'évolution dudit marqueur pour une partie au moins des cycles d'amplification,
  d. une unité de traitement comportant une mémoire et agencée pour :

    i. exprimer un paramètre ($F_0$) représentatif de la grandeur physique du marqueur d'acide(s) nucléique(s) antérieurement à un quelconque des cycles d'amplification, sur la base de mesures de grandeur physique,
    ii. estimer l'effectif initial ($N_0^{éch.}$) en acide(s) nucléique(s) d'une séquence cible présente dans l'échantillon d'intérêt à l'aide d'une loi de conversion, sur la base de paramètres contextuels,

  e. un contrôleur agencé, lorsqu'un échantillon d'intérêt est reçu sur l'élément de support, pour appliquer l'unité d'amplification et l'unité de mesure audit échantillon reçu, et pour appeler l'unité de traitement avec les mesures obtenues par ladite unité de mesure,

Dans cet appareil, les paramètres contextuels estimés avec l'unité de traitement sont des paramètres de référence sensiblement indépendants d'une partie au moins des conditions expérimentales et préenregistrés dans l'unité de traitement.

La présente invention vise également un produit programme d'ordinateur comprenant des instructions pour mettre en oeuvre le procédé sus-mentionné. Ce programme est destiné à être stocké dans une mémoire de l'unité de traitement dans l'appareil décrit ci-avant. En outre, l'invention vise un support de stockage de données, comportant des instructions pour la mise en oeuvre du procédé décrit plus haut.

D'autres avantages et caractéristiques de l'invention apparaîtront à la lecture de la description détaillée ci-après et sur les graphiques annexés sur lesquels:

- la figure 1 montre une courbe d'évolution d'une réaction en chaîne par polymérase (PCR),

- la figure 2 montre des mesures de fluorescence émise en fonction du nombre de cycle PCR,

- la figure 3 est relative à l'art antérieur pour la détermination de la population initiale en acides nucléique d'une séquence cible dans un échantillon d'intérêt et montre une courbe de régression établie à partir de la méthode des Ct,

- la figure 4 est relative à la détermination d'une grandeur physique ($F_0$) représentative de la quantité initiale d'une séquence cible présente dans un échantillon d'intérêt (art antérieur).

- la figure 5 montre une droite standard établie à partir d'échantillons de référence de même format biologique que l'échantillon à analyser,

- la figure 6 représente d'une manière schématique et fonctionnelle un appareil pour quantifier une population initiale d'un échantillon d'intérêt,

- la figure 7 montre une courbe d'exactitude d'une méthode de quantification selon l'art antérieur, et

- la figure 8 montre une courbe d'exactitude d'un exemple de réalisation de l'invention.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

On reviendra maintenant plus en détail sur les techniques permettant de quantifier la population d'acides nucléiques présente dans un échantillon d'intérêt. Par échantillon d'intérêt on défini sensiblement un échantillon à analyser. Cet échantillon d'intérêt peut donc être tout prélèvement biologique (fluide corporel notamment), susceptible de comprendre au moins une séquence cible d'acide nucléique à quantifier. Par population (ou population d'intérêt) d'acides nucléiques on défini de manière générale la quantité d'une ou plusieurs séquences cibles présentes dans un échantillon d'intérêt.

En règle générale, la population d'acides nucléiques présente dans l'échantillon d'intérêt, est trop faible pour permettre leur quantification d'une manière simple par une mesure directe. L'échantillon d'intérêt est donc soumis à un traitement d'amplification en vue d'amplifier la ou les séquences cibles à quantifier.

Le traitement d'amplification consiste d'une part à extraire la ou les séquences cibles à partir de l'échantillon d'intérêt par des méthodes d'extractions classiques connues dans l'art, et d'autre part à soumettre la ou les séquences cibles à une succession d'applications d'une réaction d'amplification. On comprendra donc que l'échantillon d'intérêt évolue dans l'avancement du traitement d'amplification. En partant d'un prélèvement biologique brut (sang ou sérum notamment), l'échantillon d'intérêt subi plusieurs phases réactionnelles (extraction, purification, lavage, etc.) impliquant divers réactifs chimiques et/ou biologiques. La composition biochimique de l'échantillon d'intérêt n'est donc pas fixe au cours de l'avancement du traitement d'amplification. L'homme du métier saura distinguer la composition biochimique au vue de la description et notamment il saura différencier entre:

- l'échantillon d'intérêt dit brut, qui défini le prélèvement biologique brut comme notamment du sang, du sérum ou un prélèvement cutané,
- échantillon d'intérêt dit extrait, qui défini échantillon après extraction et purification des acides nucléiques comprenant une ou plusieurs séquences cibles à quantifier,
- l'échantillon d'intérêt dit réactionnel, qui défini l'échantillon conditionné en vue de subir une succession d'applications d'une réaction d'amplification; l'échantillon comprend une ou plusieurs séquences cibles à quantifier, ainsi qu'un lot de réactifs nécessaire pour appliquer ladite succession d'applications d'une réaction d'amplification à l'échantillon (détaillé plus loin).

La succession d'applications d'une réaction d'amplification peut être par exemple la technique dite PCR en temps réel. Toutefois, invention n'est pas nécessairement limitée à cette technique et pourrait s'appliquer à tout traitement d'amplification (et pourrait notamment s'appliquer à la LCR "Ligase Chain Reaction").

La technique de PCR en temps réel trouve notamment son application dans le cadre du diagnostic et du suivi thérapeutique pour l'évaluation du nombre de copies d'agents pathogènes (par exemple du Virus de l'Hépatite B: VHB, du virus de l'hépatite C: VHC ou encore du virus de l'immunodéficience humaine: VIH) dans un prélèvement de fluides corporels (par exemple de sérum, plasma ou sang) d'un individu.

En PCR, la quantification s'effectue en fin de réaction, généralement sur gel d'agarose. Elle est basée sur le nombre d'acides nucléiques présents en fin de réaction d'amplification d'une séquence cible, et sur la comparaison avec une ou plusieurs séquences d'acides nucléiques présentes dans un échantillon standard ou une gamme d'échantillons standards et dont la ou les concentrations initiales sont connues.

Lorsqu'un seul échantillon standard est utilisé, il est généralement co-amplifié en parallèle avec la séquence cible à détecter. D'une façon générale, ces méthodes sont assez peu précises, et peu sensibles, d'où une quantification insatisfaisante. Le développement de la PCR en temps réel est venu apporter davantage de précision et de sensibilité.

Lors de la réalisation d'une PCR en temps réel, la quantification s'effectue au cours de la réaction d'amplification. On utilise des marqueurs par exemple des marqueurs fluorescents, spécifiques à la séquence cible à amplifier. Il existe actuellement différents systèmes faisant intervenir des marqueurs fluorescents. Citons parmi ces systèmes ceux décrits dans les documents US 5,210,015, US 5,487,972, US 5,723,591 US 5,118,801 US 5,925,517 et US 6,150,097. La fluorescence émise au cours de la réaction, et plus précisément émise après chaque cycle d'amplification évolue proportionnellement à la quantité des produits PCR, à savoir avec la quantité d'acides nucléiques ou plus précisément avec la ou les séquences cibles. Par conséquent, l'intégralité de la cinétique réactionnelle est théoriquement mesurable par cette méthode. En pratique, la mesure n'est possible qu'à partir du moment où la mesure - la fluorescence - se distingue d'un bruit de fond.

Très brièvement, par cycle d'amplification ou cycle PCR, on entend une phase au cours de laquelle l'échantillon d'intérêt subit des variations de température en présence d'un certain nombre de réactifs (dNTPs, Polymérase, amorces, etc.). Les variations de températures permettent dans un premier temps de dénaturer l'ADN double brin en cassant les structures secondaires de telle sorte que les deux brins soient dissociés. Par la suite, les variations de température vont permettre l'hybridation des amorces spécifiques d'ADN à la séquence cible, présentes dans le milieu réactionnel, et d'activer les polymérases qui vont procéder à la synthèse de nouveaux brins d'ADN (un tel brin est dit "amplicon" et est complémentaire à la séquence cible). Dans la PCR en temps réel, c'est l'ensemble des séquences cibles qui est détecté à chaque nouveau cycle par les marqueurs fluorescents.

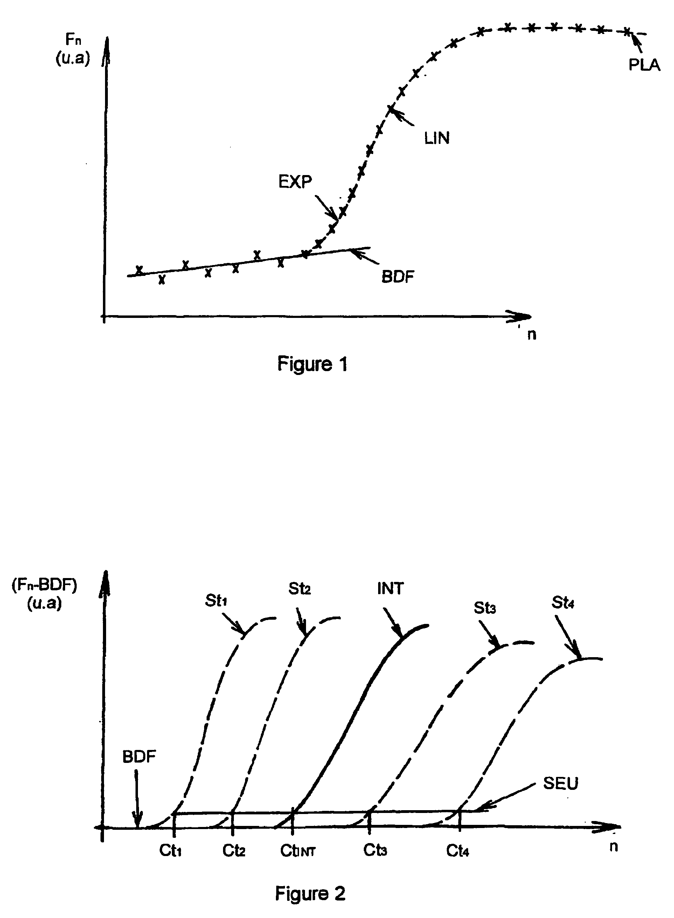

L'allure schématique d'une courbe d'amplification par PCR en temps réel est représentée sur la figure 1, avec, en abscisses des indices de cycles de PCR et, en ordonnées des quantités de fluorescence émise (en unité arbitraire [u.a]). A chaque cycle de PCR la fluorescence est mesurée.

La quantité de fluorescence $F_n$ évolue en fonction du nombre n de cycles PCR déjà effectués. On distingue successivement dans cette évolution :

- une première partie BDF où les mesures de fluorescence se confondent sensiblement avec le bruit de fond de l'appareil de mesure de fluorescence,
- une seconde partie EXP où les quantités de fluorescence mesurées croissent de façon sensiblement exponentielle,
- une troisième partie LIN où la croissance des quantités de fluorescence mesurées est sensiblement atténuée et se comporte globalement de façon sensiblement linéaire, et
- une quatrième partie PLA où les mesures de fluorescence atteignent une phase de plateau (effet de saturation).

Durant les première et seconde parties définies ci-dessus, la cinétique de la réaction répond à une loi exponentielle. Au cours de ces cycles la population d'intérêt (quantité de séquence cible) croît constamment de façon exponentielle. On définit ainsi une phase exponentielle sensiblement stationnaire de la réaction d'amplification.

Les cycles qui suivent durant les troisième et quatrième phases, n'obéissent plus à une cinétique de type exponentiel. En effet, la croissance de la population d'intérêt entre en compétition avec des phénomènes d'amortissement, notamment la limitation en dNTP, en marqueurs fluorescents et la dégradation de l'activité enzymatique de la polymérase. En définitive, on atteindra une phase de plateau. Il s'agit ici d'une phase non-stationnaire de la réaction d'amplification.

Diverses techniques ont été décrites pour estimer la quantité initiale, inconnue, d'acides nucléiques d'une séquence cible dans un échantillon d'intérêt. Le document "Mathematics of quantitative kinetic PCR and the application of standard curves", de R. G. Rutledge et C. Côté, dans la revue scientifique Nucleic Acids Research, 2003, vol.31, N°16 décrit une méthode qui consiste à utiliser plusieurs échantillons de quantités initiales connues en acides nucléiques, dits "standards", pour déterminer, par interpolation, la quantité initiale d'acides nucléiques présents dans l'échantillon d'intérêt. La figure 2 est relative à cet art antérieur.

Comme mentionné plus haut, en reportant la quantité de fluorescence mesurée $F_n$ en fonction du nombre n de cycles de PCR, on distingue une première partie BDF où les mesures de fluorescence se confondent avec un bruit de fond de l'appareil de mesure de fluorescence. Au cours de l'avancement de la réaction PCR, la fluorescence atteint un seuil de fluorescence qui se distingue du bruit de fond. Ce seuil est généralement préfixé arbitrairement par l'opérateur et se situe sensiblement au-dessus du bruit de fond.

On constate que plus la quantité initiale en acides nucléiques d'une séquence cible dans un échantillon d'intérêt est élevée, plus tôt la fluorescence se distingue du bruit de fond. Ceci est directement lié au fait que plus la quantité initiale en acides nucléiques d'une séquence cible est importante, plus tôt on atteint une quantité de produit PCR assez importante pour permettre un signal discernable du bruit de fond.

Sur la figure 2, sont représentés différents standards de quantités initiales en acides nucléiques d'une séquence connus. On comprend que la population initiale dans le standard $St_1$ est plus élevée que celle du standard $St_2$, laquelle est plus élevée que celle du standard $St_3$, et ainsi de suite. Au vu de ce qui précède, le cycle de distinction de la fluorescence intervient donc plus tôt pour le standard $St_1$ que pour le standard $St_2$ lequel intervient encore avant le cycle de distinction du standard $St_3$, et ainsi de suite.

Par conséquent, en fixant un seuil SEU de fluorescence distinct du bruit de fond BDF et commun à tous les standards, on note que le nombre $Ct_1$ de cycles PCR nécessaires au standard $St_1$ pour atteindre ce seuil préfixé est par conséquent inférieur au nombre $Ct_2$ de cycles PCR nécessaires au standard $St_2$ pour atteindre ce seuil, lequel est inférieur au nombre $Ct_3$ de cycles PCR nécessaires au standard $St_3$ pour atteindre ce seuil, etc.

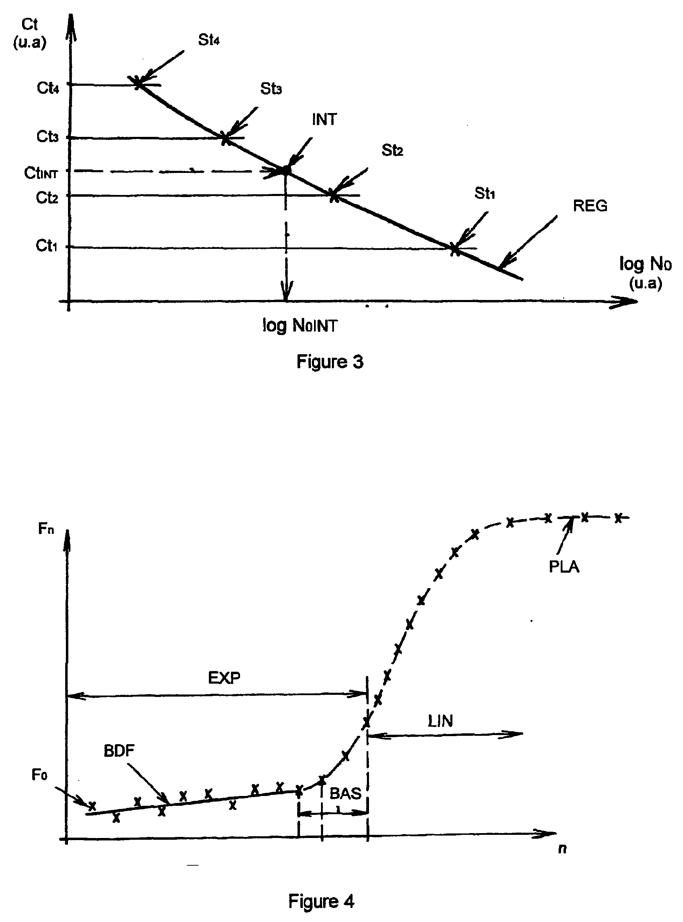

Cette observation a été mise à profit dans l'art antérieur pour établir une dépendance telle que représentée sur la figure 3 entre le nombre de cycles $Ct_1$, $Ct_2$, $Ct_3$, $Ct_4$, pour plusieurs standards d'effectifs initiaux connus, $N_0^{St1}$, $N_0^{St2}$, $N_0^{St3}$, $N_0^{St4}$. Cette dépendance, peut être visualisée au moyen d'une courbe, dite courbe de régression REG. Ainsi en reportant les cycles $Ct_1$, $Ct_2$, etc. en ordonnée, et le logarithme des effectifs initiaux $N_0^{St1}$, $N_0^{St2}$ etc. en abscisse, on obtient la courbe de régression REG. Dans le cas présent, il s'agit plus précisément d'une droite de régression. Cette droite REG servira ensuite pour la détermination de l'effectif initial en acides nucléiques d'une séquence cible dans un échantillon d'intérêt.

Ainsi, sur la figure 2 est représenté un échantillon d'intérêt d'effectif initial inconnu INT, dont on distinguera un cycle $Ct_{INT}$. Par projection du cycle $Ct_{INT}$ sur la courbe/droite de régression REG, on pourra par la suite déterminer l'effectif initial $N_0^{INT}$ de la population de l'échantillon d'intérêt (voir figure 3).

Cette méthode, bien que largement répandue, présente toutefois un certain nombre d'inconvénients dont les 3 principaux sont :

- d'être entièrement basée sur le jugé de l'opérateur pour la détermination de la valeur du seuil SEU de fluorescence se distinguant du bruit de fond. Or il est difficile de distinguer avec précision un tel seuil en raison du fait que les mesures de fluorescence dans cette région (typiquement à la transition BDF/EXP) sont très proches du bruit de fond BDF,
- de nécessiter l'utilisation de plusieurs échantillons standards dont on connaît les effectifs initiaux en acides nucléiques d'une ou plusieurs séquences, ce qui fait de cette méthode un procédé coûteux en temps et en réactifs,
- d'admettre que le rendement d'amplification de la population d'acides nucléiques est identique pour tous les échantillons standards ainsi que pour l'échantillon d'intérêt; dans le cas où l'échantillon d'intérêt contiendrait des inhibiteurs de PCR, l'estimation de l'effectif initial serait fausse, car trop basse.

Pour traiter les inconvénients précités et pour apporter d'autres avantages et notamment l'utilisation d'un seul standard, le document EP1700245 est venu introduire un procédé mis en oeuvre par des moyens informatiques de quantification absolue et/ou relative d'une population initiale d'acides nucléiques dans un échantillon d'intérêt. Dans ce procédé, l'échantillon est soumis à une succession d'applications d'une réaction d'amplification d'une population d'intérêt (une ou plusieurs séquences cibles). L'amplification peut être une PCR ou toute autre technique d'amplification, dès lors qu'il est possible de suivre la variation du rendement de la réaction correspondant à cette amplification. En effet, la déduction de la quantité initiale d'acides nucléiques dans l'échantillon utilise le fait que dans le cadre de réactions d'amplification de quantité d'acides nucléiques, le rendement bascule souvent d'une efficacité constante à une efficacité non constante. Pour définir ce basculement BAS, des mesures expérimentales représentatives d'un effectif courant de la population sont relevées au cours de l'amplification, pour finalement déduire l'effectif initial dans l'échantillon d'intérêt.

En PCR temps réel, le basculement BAS mentionné ci-dessus correspond sensiblement au basculement du rendement d'amplification observé entre la phase constante et la phase non constante, toutes deux définies plus haut. Ce basculement se situe typiquement ici, entre la phase exponentielle (partie EXP) et la phase linéaire (partie LIN). C'est au moyen de ce basculement que les inventeurs de la présente demande ont pu introduire (EP11700245) le procédé de quantification pour une population d'acides nucléiques présente dans un échantillon d'intérét mentionné ci-

dessus. Pour pouvoir déterminer avec précision le basculement BAS, il est nécessaire d'exploiter la quasi-totalité des points de mesure de la courbe d'amplification. Pour cette raison, la PCR temps réel est particulièrement adaptée pour la mise en oeuvre de ce procédé.

La figure 4 est relative à la détermination de la quantité initiale d'acides nucléiques d'une séquence cible selon le document EP1700245, fait en outre intervenir une grandeur $F_0$, qui correspond à la valeur de la fluorescence pour la population de l'échantillon d'intérêt considéré, avant toute amplification. Cette grandeur $F_0$, non mesurable, car inférieure au bruit de fond BDF, est théorique, mais sensiblement représentative de l'effectif initial. La détermination du paramètre $F_0$ est à l'origine d'une précision supérieure par rapport à l'art antérieur. En effet, la quantification de l'effectif initial d'une population d'acides nucléiques dans l'art antérieur, se fait en fixant un seuil de fluorescence dans la phase exponentielle EXP, typiquement à la sortie de la partie BDF (méthode Ct). Comme détaillé plus haut, il est difficile d'établir avec précision le seuil. Pour cette raison, la méthode décrite dans EP1700245 exploite la quasi-totalité de points de la courbe d'amplification, résultant en une précision supérieure aux méthodes connues.

À cet effet, il est en premier lieu nécessaire de formuler les hypothèses suivantes :

- durant les premiers cycles d'amplification lors de la partie exponentielle EXP, le rendement de la réaction est sensiblement constant, et
- le rendement de la réaction décroît à partir d'un certain nombre de cycles d'amplification ; cette décroissance a lieu lors des troisième et quatrième parties à savoir respectivement la partie linéaire LIN et la partie de plateau PLA.

Il est à noter que le bruit de fond est un problème expérimental puisqu'en théorie la phase exponentielle démarre dès le premier cycle. La décroissance du rendement peut avoir diverses explications telles que mentionné plus haut, et notamment la dégradation des réactifs de réaction (DNA polymérases, dNTPs, amorces, etc.) ou encore l'inhibition par des produits formés lors de la réaction.

En tout état de cause, la détermination du facteur $F_0$ nécessite d'une façon générale une transition qui passe d'un rendement constant, correspondant à une situation de croissance par amplification, vers un rendement non constant. On comprend que pour un rendement non constant, il pourra s'agir d'un rendement décroissant ou croissant. Pour la mise en oeuvre de l'invention, il suffit de détecter un basculement de rendement d'une phase constante à une phase non constante.

Pour un rendement $E_n$ de la réaction de l'amplification au cycle n et constant jusqu'au cycle k, on pourra écrire :

$$E_k = E_{k-1} = E_{k-2} = ... = Eo, \text{ où Eo est la valeur du rendement de la phase constante.}$$

L'effectif de la population d'intérêt (une ou plusieurs séquences cibles) à un cycle d'amplification est donné par la relation: $N_{n+1} = N_n + E_n.N_n$, où :

- $N_n$ est l'effectif de la population d'intérêt après une amplification d'indice n dans une succession d'amplification,
- $N_{n+1}$ est l'effectif de la population d'intérêt après une amplification suivant une amplification d'indice n lors de la succession d'amplification, et
- $E_n$ le rendement de la réaction d'amplification d'indice n.

Par récurrence, cette équation pourra s'écrire : $N_{n+1} = (1 + E_0)^{n+1}. N_0$, où $N_0$ est l'effectif initial de la population d'intérêt.

Une fois la phase constante dépassée, c'est-à-dire lorsque l'indice n+1 dépasse la région de basculement, la relation devient : $N_{n+1} = N_0 \times (1 + E_0)^{Ceep} \times fonction\ (C_{eep},\ n+1)$, où :

- l'indice $C_{eep}$ représente lui-même l'indice de basculement, proprement dit, entre la phase exponentielle et la phase linéaire; On comprendra donc que ($C_{eep}$ -1) est le dernier indice de la réaction d'amplification au cours duquel le rendement est encore constant,
- le *terme fonction ($C_{eep}$, n+1)* est une fonction particulière qui caractérise la phase non constante du rendement et qui dépend au moins de l'indice de basculement $C_{eep}$ et de l'indice courant de l'amplification n+1.

On remarquera que c'est par cette relation que sont liés l'indice de basculement $C_{eep}$ et l'effectif initial $N_0$ de la population d'intérêt.

Par la soustraction du bruit de fond, ainsi qu'un certain nombre de compensations consécutives et de relations statistiques, il sera possible de déterminer la fluorescence initiale théorique $F_0$ avant le premier cycle d'amplification. Le document EP1700245

décrit plus en détail la détermination de la grandeur $F_0$

Dans la présente description, le paramètre $F_0$ fait référence à un signal fluorescent. Toutefois, l'invention n'est aucunement limitée à une telle nature. En effet, toute autre grandeur physique mesurable avant le premier cycle d'amplification et représentative de l'effectif initial en acides nucléiques d'une séquence cible, pourra servir pour la mise en oeuvre de l'invention. Néanmoins, pour des raisons de simplicité et de compréhension, la suite de la description se limitera au mode de réalisation utilisant un signal fluorescent. Ce signal fluorescent au sens de la présente description est émis après hybridation du marqueur fluorescent à la séquence cible d'acides nucléique (méthode dite "molecular beacon"). Par conséquent, seulement la ou les séquences cibles et les amplicons, en association avec le marquer fluorescent, émettent ledit signal fluorescent.

En se basant donc sur le principe que la fluorescence émise est proportionnelle à la quantité d'acides nucléiques présents dans l'échantillon analysé, avant et pendant la réaction d'amplification, il en résulte :

$F_0 = k \times N_0^p$, avec k étant une constante réelle et p étant voisin de 1.

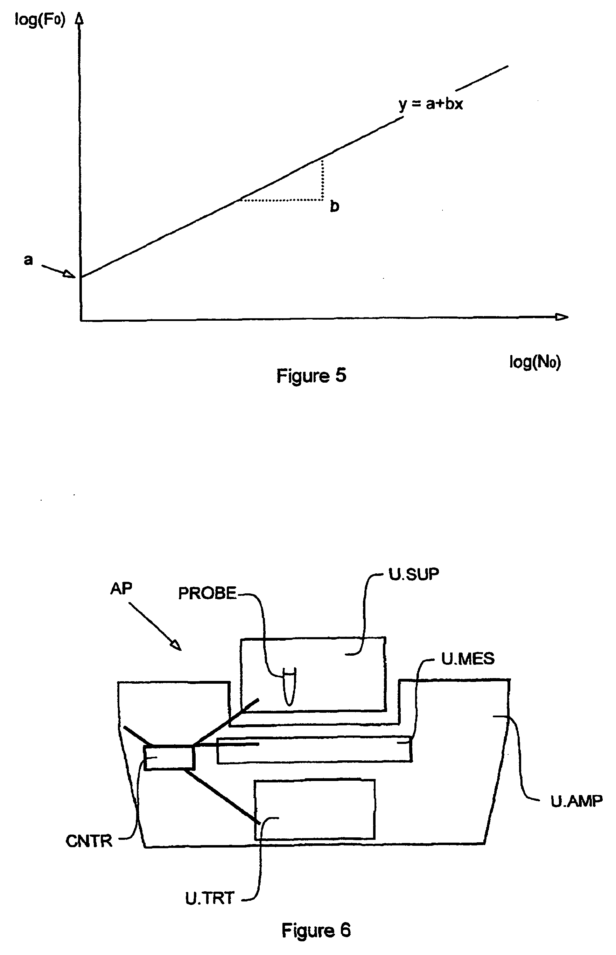

L'équation de la courbe de régression REG représentée sur la figure 5 et décrivant une relation de proportionnalité entre une concentration d'une séquence cible d'intérêt et la grandeur virtuelle $F_0$, est du type :

$$\log (F_0) = a + b \log (N_0) \qquad (F1)$$

Cette équation est de la forme y = a + bx, et décrit une droite dont les constantes a et *b*, c'est-à-dire précisément l'ordonnée à l'origine a et la pente b pourront être définies. Toutefois, dans d'autres cas l'équation de la courbe de régression REG ne suit pas une équation de droite, mais peut être par exemple du type polynomiale, sigmoïdale ou autre. Dans ce cas, le nombre de constantes peut augmenter ou diminuer selon la complexité de l'équation de la courbe REG. Pour des raisons de simplicité, le mode de réalisation décrit ici, se limite au cas d'une courbe de régression sous forme d'une droite et ainsi à la détermination de l'ordonnée à l'origine *a* et la pente *b*.

La courbe/droite REG est pré-établie à l'aide d'une gamme d'échantillons de standards (plus précisément "échantillons de référence" - détaillé plus loin), de même format biologique que l'échantillon d'intérêt. Par format biologique on entendra dans la suite de la présente description, l'ensemble:

- nature biologique de la séquence cible présente dans un échantillon, et
- conditionnement de échantillon.

La nature biologique est sensiblement définie par la séquence cible à quantifier dans un échantillon (échantillon d'intérêt ou échantillon standard/échantillon de référence). Il peut donc s'agir d'ADN ou d'ARN (génomique, plasmidique, etc.) et plus précisément de l'identité d'une séquence d'acides nucléiques (séquence complète du VHB, fragment du VHB, séquence complète du VIH, fragment du VIH etc.).

Par conditionnement d'un échantillon, on défini:

- au moins le lot de réactif essentiel pour l'exécution de la réaction d'amplification et accompagnant cet échantillon dans le milieu réactionnel, et
- le traitement qu'a subi l'échantillon depuis l'extraction jusqu'à la détermination du paramètre Fo.

L'allure de la droite REG et par conséquent des constantes a et b, sont sensiblement dépendants du format biologique de l'échantillon à analyser.

La demanderesse a découvert, non sans surprise, que des droites de régression établies à l'aide de la grandeur $F_0$ montrent une faible inter-variabilité des grandeurs a et b lorsque le format biologique de différents échantillons est identique. Cette stabilité est liée au paramètre $F_0$.

Dans la suite de la description on se référera aux échantillons standards susmentionnés en tant que échantillons de référence. Un échantillon de référence est donc: tout échantillon de même format biologique que l'échantillon d'intérêt et dont la concentration initiale en acides nucléiques d'une séquence cible est connue. Un échantillon de référence au sens de l'invention comprend donc une ou des séquences de même nature biologique que la ou les séquences cibles à quantifier. De plus, les échantillons de référence comprennent un lot de réactifs identique à celui de l'échantillon d'intérêt, et on subi sensiblement le même traitement depuis l'extraction à la détermination du paramètre $F_0$ (même conditionnement - voir plus haut).

La distinction qui est faite entre les échantillons de référence (utilisés pour l'invention) et les échantillons standards (comme utilisés pour la méthode Ct) a notamment pour objet de préciser le fait que l'opérateur manipulant les échantillons de référence est d'identité différente que celui manipulant les échantillons standards. En effet, dans la suite de la description il apparaîtra clairement que toute manipulation d'échantillon de référence est faite en usine/industrie et non par l'utilisateur final de l'invention.

Plus généralement, la demanderesse a découvert que pour la quantification d'une séquence cible d'un format biologique donné il suffit d'établir une courbe standard REG faisant intervenir une relation entre une grandeur physique représentative de l'effectif initial d'une population et la concentration connue d'au moins deux échantillons de référence. Dans le mode de réalisation décrit, la courbe standard vérifie une loi linéaire. A l'aide d'une loi de conversion, la pente et l'ordonnée à l'origine (paramètres de référence) de cette courbe permettent dans une étape ultérieure ou conjointe de déterminer l'effectif initial de la ou les séquences cibles.

Une détermination des grandeurs a et b ainsi que la détermination d'une concentration initiale d'une séquence cible, sont données plus loin dans un exemple de réalisation. Préalablement à cela, un certain nombre de termes utilisés dans la suite de la description sont définis.

Dans le mode de réalisation décrit ci-après, l'échantillon d'intérêt comprend des acides nucléiques extraits et isolés par extraction/purification à partir d'un échantillon de sérum ou de plasma humain. La ou les acides nucléiques à quantifier (également appelés séquence(s) cible(s), ou encore séquence(s) cible(s) d'intérêt) sont par exemple de l'ADN génomique viral. La ou les séquences cibles sont amplifiés et quantifiés par PCR en temps réel. Dans les exemples décrits, il s'agira plus précisément d'ADN génomique du Virus de l'hépatite B (VHB) dont la taille est d'environ 3,2 kb. Bien entendu, invention n'est aucunement limitée à cet exemple.

Par Contrôle Interne (IC) on entend, dans l'exemple de réalisation décrit, un fragment amplifié d'ADN génomique d'un organisme végétal d'effectif connu dont la séquence est hétérologue à celle de la séquence cible présente dans l'échantillon à analyser. Le contrôle interne est ajouté aux échantillons d'intérêt et au contrôle négatif (défini plus loin) dès le début de l'extraction. Il est co-amplifié avec la séquence cible. Le contrôle interne permet d'une part de s'assurer que l'extraction s'est déroulée de manière satisfaisante, et d'autre part, de contrôler l'absence d'inhibiteur dans les échantillons d'intérêt. A cet effet, il est à noter que la taille, le pourcentage en GC (Guanine/Cytosine), ainsi que l'efficacité de la réaction d'amplification, sont sensiblement identiques à ceux de la séquence cible. Dans le mode de réalisation décrit, l'IC est dilué dans une solution tampon TE (TrisHCl 10mM, EDTA 0.5mM) pH 8,3 et du Proclin™ 300. La mise en oeuvre de l'invention ne nécessite pas la prise en compte de la présence de l'IC, ceci en raison du fait qu'il n'a pas d'impact sur l'amplification de la séquence cible.

En parallèle à l'amplification de la séquence cible d'intérêt, un contrôle positif (CP) peut servir à la vérification du bon fonctionnement des réactifs spécifiques pour l'amplification de ladite séquence cible à quantifier. D'une manière générale, les réactifs dans le mélange réactionnel contenant le contrôle positif sont sensiblement identiques à ceux de l'échantillon d'intérêt. En particulier les amorces sont spécifiques à la fois à la séquence cible et au contrôle positif.

Dans le cadre d'un mode de réalisation particulier, le contrôle positif pourra aussi être utilisé en tant que, calibrateur (détaillé plus loin) unique s'il s'avère nécessaire pour la quantification de l'échantillon d'intérêt. Ce calibrateur est dénommé EQC (Calibrateur Quantitatif Externe, en Anglais « External Quantitative Calibrator »).

Dans l'exemple de réalisation décrit ici, le contrôle positif ou EQC est constitué d'un fragment amplifié d'ADN génomique viral du Virus de l'Hépatite B (VHB) dont aussi bien la quantité initiale que l'intervalle de confiance du paramètre $F_0$, sont connus. La taille ainsi que la séquence de l'EQC sont identiques à celles de la séquence cible d'intérêt. Il est amplifié avec une efficacité sensiblement identique à celle de la séquence cible dans l'échantillon d'intérêt Il s'agit d'un calibrateur externe qui n'est pas co-amplifié avec les échantillons d'intérêt, mais en parallèle de ceux-ci. Le contrôle positif ou EQC permet donc d'une part de valider les réactifs PCR spécifiques de l'échantillon d'intérêt et d'autre part peut servir, selon un second mode de réalisation de l'invention, de calibrateur pour le procédé de mesure du taux d'acides nucléiques (détaillée plus loin). Dans le mode de réalisation décrit, le contrôle positif/EQC est dilué dans du tampon TE (TrisHCl 10mM, EDTA 0.5mM) pH 8,3. Selon d'autres modes de réalisation non décrits ici, le contrôle positif/EQC pourra être dilué dans du plasma, du sérum ou autre.

Bien évidemment le mélange réactionnel peut être accompagné de tout contrôle permettant de qualifier la réaction d'amplification. Un tel contrôle peut par exemple être un Contrôle Négatif (NC). Dans l'exemple de réalisation décrit, le contrôle négatif est un échantillon de plasma humain défibrinilé négatif pour l'ADN génomique VHB contenant du Proclin™ 300. C'est-à-dire négatif pour la séquence cible d'intérêt de l'exemple de réalisation. Ce contrôle négatif est ajouté à chaque série d'extraction des acides nucléiques à partir des échantillons d'intérêt et suit le même traitement d'amplification que cet échantillon à savoir le processus d'extraction/purification et la PCR temps réel. D'une manière générale, un contrôle négatif permet de s'assurer de l'absence, de contamination dans les échantillons d'intérêt. De telles contaminations peuvent avoir lieu pendant la phase d'extraction ou lors de la préparation de la PCR temps réel. De plus, ensemble avec le contrôle interne, le contrôle négatif permet de contrôler la validité des réactifs PCR spécifiques à l'IC.

Tel qu'indiqué plus haut, le mode de réalisation décrit ici, est basé sur le principe que la fluorescence d'un échantillon d'intérêt, et plus précisément d'un échantillon d'intérêt réactionnel, est proportionnelle à la quantité d'une ou de plusieurs séquences cibles présentes dans cet échantillon, et ce aussi bien avant que pendant la réaction d'amplification. Cette constatation ressortira clairement à la lumière de l'exemple de réalisation non limitatif décrit ci-dessous.

**EXEMPLES**

L'extraction/purification ainsi que la réaction d'amplification appliqué aux des échantillons de référence et d'intérêt (PCR en temps réel) ont été effectuées selon une manière classique de l'art et de manière sensiblement identique. Le protocole expérimental utilisé est décrit ci-après.

**I. Protocole expérimental**

I-1. Echantillons de référence

Accurun 325 Contrôle positif ADN du virus de l'hépatite B - Série 700 (BBI Diagnostic - Seracare, référence A325-5723).

I-2. Extraction et purification des acides nucléiques

L'échantillon Accurun est dilué en plasma humain négatif pour la présence du VHB afin de constituer une gamme d'échantillons de référence de différentes concentrations en acides nucléiques de la séquence cible.
Chaque point de dilution est extrait en présence d'IC (dont la concentration est de 300 copies/PCR).
L'ADN viral VHB est extrait à l'aide du QIAamp DSP Virus Kit (QIAGEN, référence 60704) selon le protocole du kit.

I-3. Réaction d'amplification : PCR en temps réel.

• *Séquences oligonucléotidiques utilisées*

|  |  | **Séquences des Amorces et des Sondes** |
|---|---|---|
| **Système VHB** | Amorce 1 | 5' - GCT GAA TCC CGC GGA CGA - 3' |
|  | Amorce 2 | 5' - GTG CAG AGG TGA AGC GAA GTG - 3' |
|  | Sonde 1 | 5' FAM - CGG CAG GAG TCC GCG TAA AGA GAG GTG TGC CG -Dabcyl 3' |
| **Système IC** | Amorce 3 | 5' - GAG CCG CAG ATC CGA GCT A - 3' |
|  | Amorce 4 | 5' - GGA GTG GAA CAT AGC CGT GGT C - 3' |
|  | Sonde 2 | 5' Atto647N - TGC TGC GTC CTC CGC CGC CAC CGC TTG GGC AGC A -Dabcyl 3' |

• *Mélange de Référence :*

*Multiplex VHB / Fam Dabcyl -IC / Atto647N Dabcyl*

0.6 μM de la sonde Molecular Beacon VHB (Eurogentec), 0.6 μM des 2 amorces VHB, 0.2 μM de la sonde Molecular Beacon IC (Eurogentec), 0.3 μM des 2 amorces IC, 2.5U HotStarTaq Polymerase (QIAGEN, référence 203205), 6 mM MgCl$_2$, d(ACGU)TP 200 μM, dTTP 100 μM, 0.25U UDG, 0.3% PVP, 5% glycérol.

• *Thermoprofil sur l'appareil d'amplification (Chromo4) :*

30 min à 42°C
15 min à 95°C

$$\left.\begin{array}{l} 15 \text{ sec à } 95°C \\ 30 \text{ sec à } 55°C \\ 30 \text{ sec à } 72°C \end{array}\right\} \quad 50X$$

20°C

1-4. Interprétation des résultats.

Pour chaque échantillon (échantillons de référence ou échantillons d'intérêt), le paramètre $F_0$ est déterminé selon le procédé du document EP 1 700 145.

**II. Exemples**

II-1. Etablissement d'une courbe standard à partir d'une gamme d'échantillons de référence en utilisant la grandeur $F_0$.

Sur la figure 5 est représentée une droite standard, établie à l'échelle logarithmique, faisant apparaître la grandeur physique $F_0$ (fluorescence avant tout cycle d'amplification) en fonction de la concentration d'une séquence cible des échantillons de référence. Les points de référence servant à l'établissement de cette droite sont ceux d'une gamme d'échantillons de référence de concentrations initiales connues exprimées en UI/PCR (Unités Internationales / volume réactionnel) et dont la grandeur $F_0$ a été calculée suivant la méthode du brevet EP1700245 (voir protocole expérimental plus haut).

À des fins de précision, on procède à la détermination de $F_0$ pour 2 réplicats VHB par point de concentration (colonnes "Replicat 1" et "Replicat 2" du tableau I).

Le tableau I ci-dessous fait apparaître ces valeurs.

Tableau I

| Nombre UI ADN VHB/PCR | $F_0$ VHB | | | |
|---|---|---|---|---|
| | Replicat 1 | Replicat 2 | Moyenne $F_0$ | Ecart-type $F_0$ |
| $1.10^5$ | 4,15E-09 | 3,44E-09 | **3,79E-09** | **4,99E-10** |
| $1.10^4$ | 1,65E-10 | 1,53E-10 | **1,59E-10** | **8,58E-12** |
| 1000 | 1,09E-11 | 9,56E-12 | **1,02E-11** | **9,46E-13** |
| 100 | 7,08E-13 | 5,47E-13 | **6,28E-13** | **1,14E-13** |
| 10 | 6,23E-14 | 6,47E-14 | **6,35E-14** | **1,71E-15** |

On retiendra donc en premier lieu que, préalablement à la détermination de la quantité initiale en acides nucléiques de la séquence cible présente dans l'échantillon d'intérêt, il est nécessaire d'établir une courbe standard à partir d'au moins deux échantillons de référence de format biologique identique audit échantillon d'intérêt et de concentrations initiales en acides nucléiques connues. A partir de la courbe standard et plus précisément droite standard dans le présent exemple, l'ordonnée à l'origine et la pente (paramètres de référence *a* et *b*) peuvent être déterminées par des moyens classiques connu à l'homme du métier.

Comme décrit dans le protocole expérimental, ces mesures ont été accompagnées d'un contrôle interne IC afin de valider les réactifs et de contrôler l'étape d'extraction ainsi que l'absence d'inhibiteur. II-2. Détermination des paramètres de référence *a* et *b*.

Pour une détermination précise de *a* et de *b* c'est-à-dire respectivement de l'ordonnée à l'origine (ou intercept) et de la pente, une multitude de gammes d'échantillons de référence ont été analysées. Bien évidement, l'invention peut être réalisée sans cet affinage. Toutefois, dans l'exemple de réalisation ici présent, 22 gammes d'échantillons de référence servent à l'établissement:

- d'une valeur médiane de *a*, et
- d'une valeur médiane de *b*.

Tableau II

| Gamme d'échantillons de référence | Intercept en UI/ml (a) | Pente (b) |
|---|---|---|
| Gamme 1 | -14,989 | 1,112 |
| Gamme 2 | -15,445 | 1,220 |
| Gamme 3 | -15,234 | 1,188 |
| Gamme 4 | -15,159 | 1,169 |
| Gamme 5 | -15,258 | 1,204 |
| Gamme 6 | -15,324 | 1,196 |
| Gamme 7 | -15,582 | 1,245 |
| Gamme 8 | -15,836 | 1,295 |
| Gamme 9 | -15,449 | 1,222 |
| Gamme 10 | -15,538 | 1,229 |
| Gamme 11 | -15,452 | 1,226 |
| Gamme 12 | -14,803 | 1,052 |
| Gamme 13 | -15,075 | 1,139 |
| Gamme 14 | -14,907 | 1,103 |
| Gamme 15 | -15,031 | 1.151 |
| Gamme 16 | -15.151 | 1.185 |
| Gamme 17 | -15.113 | 1,135 |
| Gamme 18 | -14,889 | 1.091 |
| Gamme 19 | -14,742 | 1,065 |
| Gamme 20 | -15,185 | 1,136 |
| Gamme 21 | -15,337 | 1,181 |
| Gamme 22 | -14,717 | 1,029 |

Il ressort du tableau II que la variabilité des valeurs *a* et *b* (inter-variabilité) entre les différentes gammes d'échantillons de référence est faible, en raison notamment de la fiabilité et de la stabilité de la grandeur $F_0$.

C'est à partir de la médiane, aussi bien de la pente que de l'ordonnée à l'origine, que respectivement les valeurs de *a* et de *b* ont été calculés (voir tableau III). Rappelons ici que pour chaque échantillon de référence, il s'agit d'un format biologique identique à celui de l'échantillon d'intérêt. Pour un échantillon d'intérêt d'un format biologique donné, *a* et *b* sont donc des grandeurs fixes pour un lot de réactifs donné. Notons que pour l'établissement de *a* et de *b*, toute valeur statistique représentative de l'ensemble des mesures, telle que par exemple la moyenne (voir tableau III), peut être utilisée.

Tableau III

| Intercept (a) | | | Pente (b) | | |
|---|---|---|---|---|---|
| Moyenne | médiane | écart type | moyenne | mediane | écart type |
| -15,192 | -15,172 | 0,29 | 1,163 | 1,175 | 0,07 |

Les valeurs figurant dans les tableaux II et III montrent la faible variabilité de *a* et de *b.* La quantification de la population initiale $N_0$ dans un échantillon d'intérêt peut alors se faire à partir de la formule F2, déduite de la formule F1 :

$$N_0 = 10^{\frac{\log(F_0)-a}{b}} \qquad\qquad\qquad (F2)$$

En partant donc d'une source d'échantillon, similaire à un échantillon d'intérêt, mais de concentration initiale connue (ci-après dénommé échantillon test), on peut vérifier l'exactitude du test de quantification selon l'invention.

II-3. Exemple 1 - Quantification de trois échantillons test distincts.

Dans cet exemple, l'échantillon Accurun 325 Contrôle positif ADN du virus de l'hépatite B - Série 700 (BBI Diagnostic, référence A325-5723), a été dilué à trois concentrations différentes (échantillons test A, B et C) dans du plasma humain négatif pour la présence du VHB. Les trois échantillons test ont une concentration initiale en séquence cible connue (voir tableau IV)

Les acides nucléiques des échantillons test ont été extraits et purifiés en présence d'IC, à l'aide du QIAamp DSP Virus Kit (QIAGEN, référence 60704) selon le protocole du kit. Les échantillons test ont été soumis à un traitement d'amplification avec le même lot de réactifs que celui utilisé pour calculer les paramètres a et b dans l'exemple précédent.

II-3.a) Mesure des valeurs $F_0$ des trois échantillons test.

Tableau IV

| Echantillon | $F_0$ VHB | | | |
|---|---|---|---|---|
| | Réplicat 1 | Réplicat 2 | moyenne $F_0$ | Ecart-type $F_0$ |
| NC 0 UI/ml | 0 | 0 | 0 | 0 |
| Test A 19.1 UI/ml | 4,15E-14 | 2,00E-14 | **3,08E-14** | **1,52E-14** |
| Test B $1.91.10^3$ UI/ml | 3.28E-12 | 4,05E-12 | **3,67E-12** | **5,45E-13** |
| Test C $1.91.10^5$ UI/ml | 8.85E-10 | 1,12E-09 | **1,OOE-09** | **1,67E-10** |

II3.b) Quantification

Les paramètres *a* et *b* pour la souche VHB de l'exemple de réalisation ci-dessus, portent respectivement les valeurs -15,172 et 1,175 (valeur médiane, voir tableau III). En utilisant les paramètres de référence (*a* et *b*) dans la formule F2, les concentrations initiales sont déterminées/vérifiées. Les résultats figurent dans le tableau V et montrent que la quantification avec ces valeurs est très satisfaisante (erreur de quantification inférieure à un facteur deux).

Tableau V

| Echantillons | Log UI/ml | | Moyenne Log UI/ml) | Nombre UI/ml | | Moyenne UI/ml | Ecart-type UI/ml |
|---|---|---|---|---|---|---|---|
| NC 0 UI/ml | - | - | - | - | - | - | - |
| Test A **19.1 UI/ml** | 1,524 | 1,254 | **1,389** | 33 | 18 | **26** | **11** |
| Test B 1.91.103 UI/ml | 3,138 | 3,216 | **3,177** | 1375 | 1645 | **1510** | **191** |
| TestC1.91.10⁵UI/ml | 5,206 | 5,294 | **5,250** | 1.61E+05 | 1.97E+05 | **1,79E+05** | **2,53E+04** |

II-4. Exemple 2 - Exemple comparatif de quantification d'un Standard International VHB (Standard International : *WHO international standard for HB V DNA nucleic acid amplification techniques 97/746*).

Il s'agit ici d'un exemple comparatif entre la détermination de l'effectif initial d'une séquence cible présente dans un échantillon d'intérêt selon la méthode Ct d'une part et le procédé selon l'invention d'autre part. Les paramètres a et b déterminés dans l'exemple précédent ont été utilisés pour la détermination de la population initiale. L'exemple est basé sur l'utilisation du standard international dénommé WHO (*WHO international standard for HBYDNA nucleic*

*acid amplification techniques 97/746*).

Deux séries de dilutions en demi log du standard international WHO ont été réalisées en plasma humain négatif pour la présence de VHB. Chaque point de dilution a été extrait/purifié en présence d'IC, à l'aide du QIAamp DSP Virus Kit (QIAGEN, référence 60704) selon le protocole du kit. Chaque extrait a été amplifié par PCR temps réel en duplicats.

Similairement à l'Exemple 1, les valeurs de $F_0$ ont été déterminées pour chaque échantillon d'intérêt. Ensuite à partir de la formule F2, la quantification de la séquence cible contenue dans chaque échantillon est réalisée. Dans le tableau VI figurent les résultats des quantifications exprimées en échelle logarithmique.

Tableau VI

| | Méthode Ct | | | | Procédé selon l'invention | | | |
|---|---|---|---|---|---|---|---|---|
| | Standard international UI/ml [log] | | | | Standard international UI/ml [log] | | | |
| | Valeurs théoriques | Valeurs expérimentales | Moyenne | Delta de Quantification | Valeurs théoriques | Valeurs expérimentales | Moyenne | Delta de Quantification |
| **1ère série** | 6 | 6,184 | | | 6 | 6,239 | | |
| | 6 | 6,268 | 6,168 | 0,168 | 6 | 6,239 | 6,174 | 0,174 |
| **2ème série** | 6 | 6,203 | | | 6 | 6,19 | | |
| | 6 | 6,016 | | | 6 | 6,158 | | |
| **1ère série** | 5,5 | 5,606 | | | 5,5 | 5,618 | | |
| | 5,5 | 5,597 | 5,593 | 0,093 | 5,5 | 5,604 | 5,593 | 0,093 |
| **2ème série** | 5,5 | 5,543 | | | 5,5 | 5,596 | | |
| | 5,5 | 5,63 | | | 5,5 | 5,564 | | |
| **1ère série** | 5 | 4,991 | | | 5 | 5,044 | | |
| | 5 | 5,107 | 5,023 | 0,023 | 5 | 5,063 | 5,001 | 0,001 |
| **2ème série** | 5 | 5,065 | | | 5 | 4,974 | | |
| | 5 | 4,931 | | | 5 | 4,924 | | |
| **1ère série** | 4,5 | 4,465 | | | 4,5 | 4,456 | | |
| | 4,5 | 4,576 | 4,525 | 0,025 | 4,5 | 4,569 | 4,512 | 0,012 |
| **2ème série** | 4,5 | 4,43 | | | 4,5 | 4,454 | | |
| | 4,5 | 4,631 | | | 4,5 | 4,569 | | |
| **1ère série** | 4 | 3,877 | | | 4 | 3,932 | | |
| | 4 | 3,919 | 3,86 | -0,14 | 4 | 3,982 | 3,908 | -0,092 |
| **2ème série** | 4 | 3,749 | | | 4 | 3,869 | | |
| | 4 | 3,896 | | | 4 | 3,851 | | |
| **1ère série** | 3,5 | 3,474 | | | 3,5 | 3,521 | | |
| | 3,5 | 3,388 | 3,403 | -0,097 | 3,5 | 3,49 | 3,43 | -0,07 |
| **2ème série** | 3,5 | 3,343 | | | 3,5 | 3,353 | | |
| | 3,5 | 3,409 | | | 3,5 | 3,354 | | |

(suite)

| | Méthode Ct | | | | Procédé selon l'invention | | | |
|---|---|---|---|---|---|---|---|---|
| | Standard international UI/ml [log] | | | | Standard international UI/ml [log] | | | |
| | Valeurs théoriques | Valeurs expérimentales | Moyenne | Delta de Quantification | Valeurs théoriques | Valeurs expérimentales | Moyenne | Delta de Quantification |
| 1ère série | 3 | 3,07 | 2.989 | -0,011 | 3 | 3,099 | 3,052 | 0,052 |
| | 3 | 2,94 | | | 3 | 3,094 | | |
| 2ème série | 3 | 2,959 | | | 3 | 3,002 | | |
| | 3 | 2,987 | | | 3 | 3,012 | | |
| 1ère série | 2,5 | 2,327 | 2,284 | -0,216 | 2,5 | 2,581 | 2,511 | 0.011 |
| | 2,5 | 2,213 | | | 2,5 | 2,475 | | |
| 2ème série | 2,5 | 2,252 | | | 2,5 | 2,47 | | |
| | 2,5 | 2,341 | | | 2.5 | 2,517 | | |
| 1ère série | 2 | 1.98 | 1,91 | -0,09 | 2 | 2.263 | 2.174 | 0,174 |
| | 2 | 1.868 | | | 2 | 2,132 | | |
| 2ème série | 2 | 1,854 | | | 2 | 2.122 | | |
| | 2 | 1,938 | | | - 2 | 2,178 | | |
| 1ère série | 1,5 | 1,417 | 1.379 | -0,121 | 1,5 | 1.793 | 1,767 | 0,267 |
| | 1,5 | 1,417 | | | 1.5 | 1,775 | | |
| 2ème série | 1,5 | 1,292 | | | 1.5 | 1.681 | | |
| | 1,5 | 1,39 | | | 1,5 | 1.819 | | |
| 1ère série | 1 | 1,089 | 0,714 | -0,286 | 1 | 1.396 | 1,128 | 0,128 |
| | 1 | 0.413 | | | 1 | 0.997 | | |
| 2ème série | 1 | - 0,639 | | | 1 | 0.992 | | |
| | 1 | | | | 1 | | | |
| 1ère série | 0.5 | -0,199 | -0.199 | -0.699 | 0.5 | 0,727 | 0,727 | 0,227 |
| | 0,5 | | | | 0.5 | | | |
| 2ème série | 0,5 | | | | 0,5 | | | |
| | 0,5 | | | | 0,5 | | | |

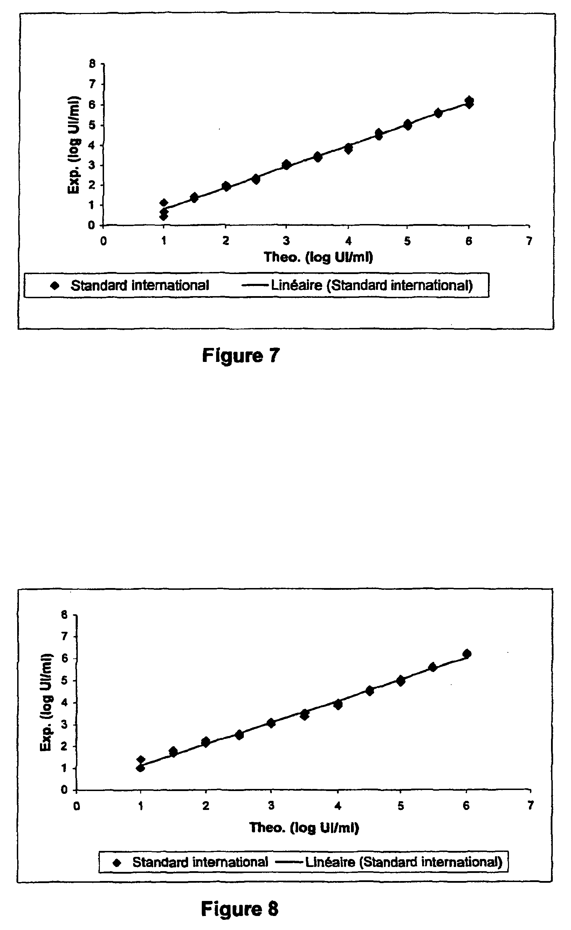

Les figures 7 et 8 montrent l'exactitude, respectivement de la méthode Ct et du procédé selon l'invention, avec en abscisses les valeurs théoriques (en Log UI/ml) et en ordonnées les valeurs expérimentales (en Log UI/ml). Les équations des droites des figures 7 et 8 sont respectivement:

$$y = -0,2966 + 1,0682x \quad ; \text{ pour la méthode Ct, et}$$
$$y = 0,1305 + 0,983x \quad ; \text{ pour le procédé selon l'invention.}$$

La quantification selon l'invention est équivalente en termes de précision à la méthode Ct, et présente tous les avantages énumérés dans la présente description avec notamment la simplicité de quantification grâce au paramètre $F_0$.

II-4. Exemple 3 - Quantification d'échantillons cliniques.

25 échantillons cliniques, provenant de patients infectés par le VHB, quantifiés préalablement par un kit commercial de quantification du virus de l'hépatite B (kit utilisant la méthode Ct) ont été testés selon la méthode de quantification de l'invention. L'extraction, la purification et le traitement d'amplification sont ceux du protocole expérimental décrit plus haut.

Chaque séquence cible dans chacun des échantillons cliniques a été amplifiée avec le même lot de réactifs que celui utilisé pour déterminer les paramètres a et b dans les exemples précédents.

Similairement à l'Exemple 1, les valeurs de $F_0$ ont été déterminées pour chaque échantillon d'intérêt. Ensuite à partir de la formule F2, la quantification de la population initiale des échantillons a été réalisée. Dans le tableau VII figurent les résultats des quantifications.

| Echantillons cliniques | | | | | |
|---|---|---|---|---|---|
| N° échantillons | Quantification *Roche Cobas TaqMan* | | Quantification selon l'invention | | |
| | Log UI/ml | UI/ml | F0 VHB | Log UI/ml | UI/ml |
| 1 | **1,362** | 23 | 5,10E-14 | **1,600** | 40 |
| 2 | **3,856** | 7176 | 1,41E-11 | **3,676** | 4744 |
| 3 | **3,268** | 1855 | 3,20E-12 | **3,129** | 1345 |
| 4 | **3,403** | 2530 | 6,05E-12 | **3,364** | 2314 |
| 5 | **2,312** | 205 | 3,20E-13 | **2,277** | 189 |
| 6 | **2,509** | 323 | 4,88E-13 | **2,434** | 271 |
| 7 | **2,938** | 866 | 1,31E-12 | **2,798** | 628 |
| 8 | **2,303** | 201 | 2,99E-13 | **2,253** | 179 |
| 9 | **2,620** | 417 | 6,52E-13 | **2,541** | 347 |
| 10 | **3,561** | 3637 | 1.40E-11 | **3,674** | 4726 |
| 11 | **2,958** | 908 | 3,46E-12 | **3,157** | 1436 |
| 12 | **2,149** | 141 | 2,51E-13 | **2,188** | 154 |
| 13 | **4,111** | 12915 | 3,98E-11 | **4,060** | 11479 |
| 14 | **3,208** | 1613 | 1,85E-12 | **2,927** | 845 |
| 15 | **3,410** | 2572 | 5,75E-12 | **3,346** | 2216 |
| 16 | **4,098** | 12528 | 3,16E-11 | **3,975** | 9432 |
| 17 | **3,548** | 3533 | 5,03E-12 | **3,296** | 1976 |
| 18 | **2,923** | 838 | 2,57E-12 | **3,048** | 1117 |
| 19 | **3,719** | 5231 | 8,26E-12 | **3,479** | 3016 |
| 20 | **2,286** | 193 | 1,43E-13 | **1,981** | 96 |
| 21 | **2,959** | 910 | 2,27E-12 | **3,001** | 1003 |
| 22 | **5,282** | 191392 | 2,30E-09 | **5,559** | 362265 |
| 23 | **2,773** | 593 | 1,43E-12 | **2,832** | 679 |
| 24 | **2,624** | 421 | 7,22E-13 | **2,578** | 379 |
| 25 | **2,407** | 255 | 4,66E-13 | **2,417** | 261 |

Les résultats obtenus selon le procédé de l'invention sur les échantillons cliniques sont équivalents aux quantifications obtenues avec un kit commercial de référence, tout en présentant les avantages résultant du procédé selon l'invention. L'exemple précédent souligne que invention répond aux exigences réglementaires et à l'étroite justesse de quantification requise pour des tests médicaux, tels que la quantification d'acides nucléiques viraux dans un échantillon de patient

Les exemples précédents illustrent la simplicité pour un opérateur final à réaliser une quantification d'une population d'acides nucléiques au moyen du procédé selon l'invention. En effet, au moins deux étapes, à savoir : établir une courbe standard et déterminer les paramètres constants de l'équation de la courbe standard, sont exécutées en usine/industrie et préenregistrées dans un appareil PCR selon l'invention. L'opérateur final profitera ainsi d'une simplicité d'utilisation pour mettre en oeuvre la quantification d'une ou plusieurs séquences cibles présents dans un échantillon d'intérêt. Principalement et grâce aux paramètres préenregistrés, l'opérateur contournera les deux étapes précitées. Il en suit un gain de temps par rapport aux procédés de l'état de la technique. De plus, les exemples montrent clairement la robustesse du procédé. Cette robustesse est due à l'utilisation de $F_0$.

En pratique, l'opérateur n'aura qu'à soumettre l'échantillon d'intérêt au traitement d'amplification c'est-à-dire extraction/purification des acides nucléiques de l'échantillon d'intérêt brut afin d'obtenir un échantillon d'intérêt extrait, puis ajout à ce dernier d'un lot de réactifs pour obtenir un échantillon d'intérêt réactionnel, et finalement appliquer l'échantillon d'intérêt réactionnel dans une unité de support d'un appareil d'amplification. L'appareil d'amplification déterminera la valeur de $F_0$ (selon le procédé décrit dans EP1700245) et au moyen de cette valeur $F_0$ et ensemble avec les paramètres préenregistrés (constantes d'équation de courbe standard) déterminera à lui seul la quantité initiale en acides nucléiques d'une ou plusieurs séquences cibles présentes dans l'échantillon d'intérêt. Tout ceci fait que l'invention présente un grand intérêt économique.

Bien entendu, les valeurs de *a* et de *b* varient en fonction de la nature biologique de la séquence cible. Comme détaillé plus haut, ces valeurs varient également en fonction du lot de réactifs appliqué à l'échantillon d'intérêt pour la réalisation de la réaction d'amplification. Néanmoins, pour un lot de réactifs donné, les valeurs a et b sont stables dans le temps pendant toute la durée de validité du lot c'est-à-dire jusqu'à sa date de péremption. Ceci est synonyme d'une grande fiabilité pour l'opérateur final.

Plus généralement, les valeurs de *a* et de *b* sont indépendantes de:

- l'appareil utilisé pour le traitement d'amplification, (en particulier d'un même modèle commercial),
- du type d'appareil utilisé, (on peut donc utiliser tout type de modèle commercial, notamment l'appareil dénommé "Chromo 4" de la société Bio-Rad ou l'appareil dénommé "light cycler" de la société Roche),
- du site de manipulation (transportabilité de l'appareil),
- de l'opérateur réalisant les manipulation techniques,
- de la validité du lot de réactifs,
- composants individuels utilisés dans le lot de réactifs (on peut donc utiliser tout type de polymérase, de dNTP, etc...),
- mode d'extraction des acides nucléiques de la séquence cible.

L'utilisation de la relation entre $F_0$ et $N_0$ semble être à l'origine de la faible variabilité et de la stabilité des valeurs *a* et de *b.*

Grâce à ces avantages et à la justesse de la quantification, l'invention répond aux exigences réglementaires requises pour des tests médicaux, tels que la quantification d'acides nucléiques viraux dans un échantillon d'intérêt de patient.

Selon ce premier mode de réalisation de l'invention et contrairement à l'état de l'art, il ne sera donc plus nécessaire pour l'opérateur d'utiliser une gamme de standards, pour la détermination de la population initiale de l'échantillon d'intérêt à quantifier. En effet, c'est grâce aux paramètres de référence prédéterminés et préenregistrés, notamment *a* et *b* dans les exemples de réalisation, pour un format biologique donné que l'on détermine la population initiale d'un échantillon.

Selon un autre mode de réalisation, un seul calibrateur ou EQC (calibrateur externe), qui peut être le contrôle positif, peut être utilisé pour procéder à une correction des paramètres de référence préenregistrés, lorsque ceux-ci présentent une variabilité de la précision de la détermination de la population initiale.

La correction utilise le paramètre $F_0$. Plus précisément, on détermine lors d'une étape préalable à l'étape d'estimation de l'effectif initial de la population d'intérêt, le $F_0$ de consigne (ci-après: $F_0^{EQC}$) de l'EQC. Cette détermination a donc lieu avant l'application de ladite loi de conversion.

Ensuite, on procède à une simple mesure comparative, entre la valeur $F_0$ de l'EQC mesurée dans l'appareil (ci-après: $F_0^{EQCmesuré}$), et la valeur $F_0^{EQC}$ connue dans un intervalle de confiance déterminé en usine/industrie. On pourra alors établir une relation correctrice de proportionnalité pour palier l'écart des valeurs. Les paramètres contextuels à l'étape d. du procédé selon l'invention, peuvent donc comporter des paramètres de correction.

Dans des modes de réalisation en variante, la correction peut être basée sur d'autres coefficients, avec des relations de proportionnalité non linéaires.

Plus généralement il suffira donc de déterminer une grandeur physique de référence à partir d'un calibrateur, et de calibrer l'appareil sur cette grandeur.

Le procédé décrit ci-dessus est de préférence mis en oeuvre avec un unique appareil équipé d'unités distinctes ou conjointes apte à réaliser les étapes dudit procédé et notamment capable de réaliser l'amplification d'une ou plusieurs séquences cibles présentes dans l'échantillon d'intérêt comme décrit plus haut.

On se réfère à la figure 6 sur laquelle est représenté d'une manière schématique et fonctionnelle, un appareil AP pour quantifier une population initiale d'un échantillon d'intérêt. Cet appareil AP, comporte une unité de support U.SUP capable de recevoir au moins un échantillon d'intérêt réactionnel PROBE. Cet échantillon PROBE est soumis à un traitement d'amplification comportant une succession d'applications d'une réaction d'amplification au moyen d'une unité d'amplification U.AMP. L'unité d'amplification U.AMP peut donc être équipée de moyens de chauffage (non représentés) en vue d'appliquer à l'échantillon réactionnel PROBE et plus précisément, à au moins une séquence cible présente dans l'échantillon réactionnel PROBE, une réaction d'amplification (comme la PCR en temps réel) durant laquelle on poursuit la cinétique réactionnelle. A cet effet, l'appareil AP comprend une unité de mesure U.MES pour mesurer une grandeur physique représentative de l'évolution d'au moins un marqueur présent dans l'échantillon d'intérêt réactionnel. Le ou les marqueurs sont respectivement spécifiques à la ou les séquences cibles. Cette grandeur physique est de préférence la fluorescence. De plus, l'appareil est équipé d'une unité de traitement U-TRT qui est agencé pour calculer le paramètre Fo (selon le procédé du document EP1700245) et estimer l'effectif initial en acide nucléique de l'échantillon d'intérêt. L'unité de traitement U-TRT peut être un ordinateur. L'unité de traitement U-TRT est munie d'une mémoire capable de stocker des données informatiques (mémoire permanente, vive ou type RAM). Les données informatiques peuvent notamment comprendre les instructions pour la mise en oeuvre du procédé de quantification de l'invention. Optionnellement l'unité de traitement U-TRT peut être munie de moyens de transfert de données aptes à recevoir un support de stockage (notamment: disquette, CD-ROM, clé USB). L'unité de traitement U-TRT peut donc subir des mises a jour. L'application de l'unité d'amplification U.AMP et de l'unité de mesure U.MES à l'échantillon d'intérêt réactionnel PROBE reçu sur l'élément de support, ainsi que appeler l'unité de traitement U-TRT, est assurée par un contrôleur CNTR. La fonction du contrôleur CNTR peut être commandé par un ordinateur ou par un opérateur.

L'invention vise également un support de stockage de donnés capable de coopérer avec l'unité de traitement U-TRT et comportant des instructions pour la mise en oeuvre du procédé de quantification de l'invention.

L'invention est décrite ci-dessus en référence à la PCR en temps réel. Elle n'est pas limitée à cette technique et pourrait s'appliquer à d'autres moyens d'amplification d'acides nucléiques. Elle pourrait par exemple s'appliquer à la LCR. Plus précisément on utilisera toute technique d'amplification dès lors qu'il est possible de suivre la variation du rendement de la réaction correspondant à cette amplification.

**Revendications**

1. Procédé de mesure du taux d'acides nucléiques d'une séquence cible dans un échantillon d'intérêt, comprenant les étapes de :

   a. soumettre l'échantillon d'intérêt à un traitement d'amplification, avec un lot de réactifs comprenant au moins un marqueur d'acide(s) nucléique(s) spécifique à ladite séquence cible, ledit traitement d'amplification comportant des cycles d'amplification successif, i; de 1 à n,
   b. mesurer une grandeur physique $F_i$ représentative de l'évolution du marqueur pour une partie au moins des cycles d'amplification,
   c. exprimer un paramètre $F_0$ représentatif de la grandeur physique du marqueur d'acide(s) nucléique(s) avant tout cycle d'amplification, en utilisant les mesures effectuées à l'étape b.,
   d. estimer l'effectif initial $N_0^{éch}$ en acide(s) nucléique(s) de la séquence cible à l'aide d'une loi de conversion, comportant des paramètres contextuels, et appliquée audit paramètre $F_0$ exprimé à l'étape c.,

   **caractérisé en ce que** :

   - à l'étape d., lesdits paramètres contextuels sont des paramètres de référence préenregistrés, ces paramètres étant sensiblement indépendants d'une partie au moins des conditions expérimentales, et étant déterminés à l'avance à partir du comportement d'au moins un-échantillon de référence de même format biologique que l'échantillon d'intérêt et d'effectif initial $N_0^{réf}$ connu en acide(s) nucléique(s).

2. Procédé selon la revendication 1, **caractérisé en ce que** la loi de conversion à l'étape d., est appliquée directement, sans nécessiter de correction par l'exécution en parallèle des étapes a., b. et c. pour au moins un calibrateur.

**3.** Procédé selon la revendication 1, **caractérisé en ce que** l'étape d. comprend en outre une correction de l'effectif initial $N_0^{éch}$ estimé, cette correction étant fondée sur une exécution à l'avance des étapes a, b. et c. pour au moins un calibrateur EQC d'effectif initial en acide(s) nucléique(s) et de paramètre $F_0^{EQC}$ représentatif de la grandeur physique du marqueur d'acide(s) nucléique(s) avant tout cycle d'amplification connus.

**4.** Procédé selon la revendication 3, **caractérisé en ce que** la correction de l'effectif initial $N_0^{éch}$ estimé comprend les étapes de :

- établir une loi correctrice entre:

• ledit paramètre $N_0^{EQC}$ représentatif de la grandeur physique du marqueur d'acide(s) nucléique(s) avant tout cycle d'amplification connu d'une part et,
• le paramètre $N_0^{EQCmesuré}$ représentatif de la grandeur physique du marqueur d'acide(s) nucléique(s) avant tout cycle d'amplification effectivement exprimé du calibrateur,

- appliquer ladite loi correctrice à ladite loi de conversion comportant les paramètres contextuels de l'étape d., pour l'estimation dudit effectif initial $N_0^{éch}$ en acide(s) nucléique(s) de la séquence cible, présent(s) dans l'échantillon d'intérêt.

**5.** Procédé selon l'une des revendications 3 ou 4, **caractérisé en ce que** ledit calibrateur EQC d'effectif initial connu se fonde sur une substance biologique propre à former un contrôle positif pour l'échantillon d'intérêt

**6.** Procédé selon l'une des revendications précédentes, **caractérisé en ce que** l'expression du paramètre $F_0$ fait intervenir, au moins :

- un paramètre relatif au basculement entre une première phase de rendement d'amplification constant et une seconde phase de rendement d'amplification non-constant,
- un paramètre relatif au rendement d'amplification constant lors de ladite première phase, et
- un paramètre relatif au rendement d'amplification non-constant lors de ladite seconde phase.

**7.** Procédé selon l'une des revendications précédentes, **caractérisé en ce que** le marqueur d'acides nucléiques est un marqueur fluorescent.

**8.** Procédé selon l'une des revendications précédentes, **caractérisé en ce que** l'échantillon d'intérêt comprend un prélèvement biologique susceptible de comprendre un agent pathogène.

**9.** Procédé selon l'une des revendications précédentes, **caractérisé en ce que** la réaction d'amplification est une Réaction en Chaîne par Polymérase, PCR, en temps réel.

**10.** Procédé selon l'une des revendications précédentes, **caractérisé en ce que** lesdits paramètres de référence sont indépendants d'au moins un des éléments du groupe de conditions expérimentales suivantes : appareil utilisé, type d'appareil utilisé, opérateur, période de validité du lot de réactifs, mode d'extraction des acides nucléiques de la séquence cible.

**11.** Procédé selon l'une des revendications précédentes, **caractérisé en ce que** l'échantillon d'intérêt comprend plusieurs séquences cibles distinctes.

**12.** Appareil (AP) pour mesurer le taux d'acides nucléiques d'au moins une séquence cible dans un échantillon d'intérêt, comprenant :

a. un élément de support (U.SUP) pour supporter au moins un échantillon (PROBE) comprenant un lot de réactifs et la séquence cible avec au moins un marqueur d'acide(s) nucléique(s) spécifique à ladite séquence cible,
b. une unité d'amplification (U:AMP) pour soumettre l'échantillon d'intérêt à un traitement d'amplification comportant des cycles d'amplification successifs, i ; de 1 à n,
c. une unité de mesure (U.MES) pour mesurer une grandeur physique $F_i$ représentative de l'évolution dudit marqueur pour une partie au moins des cycles d'amplification,
d. une unité de traitement (U.TRT) comportant une mémoire et agencée pour:

iii. exprimer un paramètre $F_0$ représentatif de la grandeur physique du marqueur d'acide(s) nucléique(s) antérieurement à un quelconque des cycles d'amplification, sur la base de mesures de grandeur physique,

iv. estimer l'effectif initial $N_0^{\text{éch}}$ en acide(s) nucléique(s) d'une séquence cible présente dans l'échantillon d'intérêt à l'aide d'une loi de conversion, sur la base de paramètres contextuels, et appliquée audit paramètre $F_0$

e. un contrôleur (CNTR) agencé, lorsqu'un échantillon d'intérêt (PROBE) est reçu sur l'élément de support (U.SUP), pour appliquer l'unité d'amplification (U.AMP) et l'unité de mesure (U.MES) audit échantillon (PROBE) reçu, et pour appeler l'unité de traitement (U.TRT) avec les mesures obtenues par ladite unité de mesure (U.MES),

**caractérisé en ce que** lesdits paramètres contextuels sont des paramètres de référence sensiblement indépendants d'une partie au moins des conditions expérimentales et préenregistrés dans l'unité de traitement (U.TRT), et étant déterminés à l'avance à partir du comportement d'au moins un échantillon de référence de même format biologique que l'échantillon d'intérêt et d'effectif initial $N_0^{\text{réf}}$ connu en acide(s) nucléique(s),

13. Produit programme d'ordinateur comportant des instructions pour la mise en oeuvre du procédé selon l'une des revendications 1 à 11, et destiné à être stocké dans la mémoire de unité de traitement (U.TRT) dans l'appareil (AP) selon la revendication 12:

14. Support de stockage de données, **caractérisé en ce qu'**il comporte des instructions pour la mise en oeuvre du procédé selon l'une des revendications 1 à 11.

## Claims

1. Method for measuring the amount of nucleic acids of a target sequence in a sample of interest, comprising the steps of:

   a. subjecting the sample of interest to an amplification treatment, with a batch of reagents comprising at least one nucleic acid(s) label specific for said target sequence, said amplification treatment comprising successive amplification cycles, i; from 1 to n,

   b. measuring a physical quantity $F_i$ representative of the evolution of the label for at least a part of the amplification cycles,

   c. expressing a parameter $F_0$ representative of the physical quantity of the nucleic acid(s) label before any amplification cycle, using the measurements carried out in step b.,

   d. estimating the initial number $N_0^{\text{sam}}$ of nucleic acid(s) of the target sequence using a conversion law, comprising contextual parameters, and applied to said parameter $F_0$ expressed in step c.,

   **characterized in that**:

   - in step d., said contextual parameters are pre-recorded reference parameters, these parameters being substantially independent of at least a part of the experimental conditions, and being determined in advance based on the behaviour of at least one reference sample of the same biological format as the sample of interest and of known initial number $N_0^{\text{ref}}$ of nucleic acid(s).

2. Method according to Claim 1, **characterized in that** the conversion law in step d. is applied directly, without the need for correction by the execution in parallel of steps a., b. and c. for at least one calibrator.

3. Method according to Claim 1, **characterized in that** step d. also comprises a correction of the estimated initial number $N_0^{\text{sam}}$, this correction being based on steps a., b. and c. being carried out in advance for at least one calibrator EQC of known initial number of nucleic acid(s) and of known parameter $F_0^{\text{EQC}}$ representative of the physical quantity of the nucleic acid(s) label before any amplification cycle.

4. Method according to Claim 3, **characterized in that** the correction of the estimated initial number $N_0^{\text{sam}}$ comprises the steps of:

   - establishing a corrective law between:

• said known parameter $F_0^{EQC}$ representative of the physical quantity of the nucleic acid(s) label before any amplification cycle, firstly, and
• the effectively expressed parameter $F_0^{EQCmeasured}$ representative of the physical quantity of the nucleic acid(s) label before any amplification cycle, of the calibrator,

- applying said corrective law to said conversion law comprising the contextual parameters of step d., in order to estimate said initial number $N_0^{sam}$ of nucleic acid(s) of the target sequence, present in the sample of interest.

5. Method according to any of claims 3 or 4, **characterized in that** said calibrator EQC of known initial number is based on a biological substance substance able to form a positive control for the sample of interest.

6. Method according to any one of the preceeding claims, **characterized in that** the expression of the parameter $F_0$ involves at least:

- one parameter relating to the switch between a first phase of constant amplification yield and a second phase of non-constant amplification yield,
- one parameter relating to the constant amplification yield during said first phase, and
- one parameter relating to the non-constant amplification yield during said second phase.

7. Method according to any one of the preceeding claims, **characterized in that** the nucleic acids label is a fluorescent label.

8. Method according to any one of the preceeding claims, **characterized in that** the sample of interest comprises a biological specimen that may comprise a pathogenic agent.

9. Method according to any one of the preceeding claims, **characterized in that** the amplification reaction is a real time polymerase chain reaction (PCR).

10. Method according to any one of the preceeding claims, **characterized in that** said reference parameters are independent of at least one of the elements of the group of following experimental conditions: apparatus used, type of apparatus used, operator, period of validity of the batch of reagents, method of extraction of the nucleic acids of the target sequence.

11. Method according to Claim 1, **characterized in that** the sample of interest comprises several distinct target sequences.

12. Apparatus (AP) for measuring the amount of nucleic acids of at least one target sequence in a sample of interest, comprising:

a. a carrier element (U.SUP) for carrying at least one sample (PROBE) comprising a batch of reagents and the target sequence with at least one nucleic acid(s) label specific for said target sequence,
b. an amplification unit (U:AMP) for subjecting the sample of interest to an amplification treatment comprising successive amplification cycle i; from 1 to n,
c. a measuring unit (U.MES) for measuring a physical quantity $F_i$ representative of the evolution of said label for at least a part of the amplification cycles,
d. a processing unit (U.TRT) comprising a memory and organized so as:

i. to express a parameter $F_0$ representative of the physical quantity of the nucleic acid(s) label prior to any of the amplification cycles, on the basis of measurements of physical quantity,
ii. to estimate the initial number $N_0^{sam}$ of nucleic acid(s) of a target sequence present in the sample of interest using a conversion law, on the basis of contextual parameters, and applied to said parameter $F_0$,

e. a controller (CNTR) organized so that, when a sample of interest (PROBE) is received on the carrier element (U.SUP), it applies the amplification unit (U.AMP) and the measuring unit (U.MES) to said sample (PROBE) received, and calls up the processing unit (U.TRT) with the measurements obtained by said measuring unit (U.MES),

**characterized in that** said contextual parameters are reference parameters which are substantially independent of at least a part of the experimental conditions and are pre-recorded in the processing unit (U.TRT), and being determined in advance based on the behaviour of at least one reference sample of the same biological format as the sample of interest and of known initial number $N_0^{ref}$ of nucleic acid(s).

**13.** Computer program product comprising instructions for implementing the method according to any of claims 1 to 11, and intended to be stored in the memory of the processing unit (U.TRT) in the apparatus (AP) according to Claim 12.

**14.** Data storage support, **characterized in that** it comprises instructions for implementing the method according to any of claims 1 to 11.

**Patentansprüche**

**1.** Verfahren zum Messen der Menge an Nukleinsäuren einer Zielsequenz in einer interessierenden Probe, das die folgenden Schritte aufweist:

a. Unterwerfen der interessierenden Probe einer Vervielfältigungsbehandlung, mit einer Reagenz-Charge, die wenigstens einen Marker von Nukleinsäure(n) aufweist, der spezifisch für die Zielsequenz ist, wobei die Vervielfältigungsbehandlung aufeinandertolgende Vervielfältigungszyklen, i; von 1 bis n, aufweist,
b. Messen einer physikalischen Größe $F_j$, die die Entwicklung des Markers für wenigstens einen Teil der Vervielfältigungszyklen repräsentiert,
c. Ausdrücken eines Parameters $F_0$, der repräsentativ ist für die physikalische Größe des Markers von Nukleinsäure(n) vor jeglichem Vervielfältigungszyklus, unter Verwendung der Messungen, die beim Schritt b durchgeführt wurden,
d. Abschätzen des Ausgangsumfangs $N_0^{éch}$ von Nukleinsäure(n) der Zielsequenz mit Hilfe einer Umwandlungsregel, die kontextabhängige Parameter aufweist und auf der Parameter $F_0$ angewendet wird, der im Schritt c. ausgedrückt wurde,

**dadurch gekennzeichnet, dass**:

- beim Schritt d. die kontextabhängigen Parameter vorgespeicherte Referenzparameter sind, wobei diese Parameter im Wesentlichen unabhängig von wenigstens einem Teil der experimentellen Bedingungen sind, und vorab bestimmt wurden, ausgehend von dem Verhalten wenigstens einer Referenzprobe vom selben biologischen Format wie die interessierende Probe und von bekannter Ausgangsmenge $N_0^{réf}$ an Nukleinsäure(n).

**2.** Verfahren nach Anspruch 1, **dadurch gekennzeichnet, dass** die Umwandlungsregel im Schritt d. direkt, ohne eine Korrektur zu erfordern, durch paralleles Ausführen der Schritte a., b. und c. für wenigstens einen Kalibrator angewendet wird.

**3.** Verfahren nach Anspruch 1, **dadurch gekennzeichnet, dass** der Schritt d. darüber hinaus eine Korrektur der geschätzten Ausgangsmenge $N_0^{éch}$ aufweist, wobei diese Korrektur gestützt ist auf ein vorheriges Ausführen der Schritte a., b. und c. für wenigstens einen Kalibrator EQC dessen Ausgangsmenge an Nukleinsäure(n) und dessen Parameter $F_0^{EQC}$ repräsentativ ist für die physikalische Größe des Markers von Nukleinsäure(n) vor jeglichem bekannten Vervielfältigungszyklus.

**4.** Verfahren nach Anspruch 3, **dadurch gekennzeichnet, dass** die Korrektur der Ausgangsmenge $N_0^{éch}$, folgende Schritte aufweist:

- Aufstellen einer Umwandlungsregel zwischen:

• dem Parameter $F_0^{EQC}$, der repräsentativ ist für die bekannte physikalische Größe des Markers von Nukleinsäure(n) vor jeglichem Vervielfältigungszyklus einerseits, und
• dem Parameter $F_0^{EQCgemessen}$, der repräsentativ ist für die vom Kalibrator tatsächlich ausgedrückte physikalische Größe des Markers von Nukleinsäure(n) vor jeglichem Vervielfältigungszyklus,

- Anwenden der Korrekturregel auf die Umwandlungsregel, die die kontextabhängige Parameter des Schrittes d. aufweist, für das Schätzen der Ausgangsmenge $N_0^{éch}$ an Nukleinsäure(n) der Zielsequenz, die in der interessierenden Probe vorhanden ist (sind).

5. Verfahren nach Anspruch 3 oder 4, **dadurch gekennzeichnet, dass** der Kalibrator EQC von bekannter Ausgangsmenge auf einer biologischen Substanz basiert, die geeignet ist, eine Positivkontrolle für die interessierende Probe zu bilden.

6. Verfahren nach einem der vorhergehenden Ansprüche, **dadurch gekennzeichnet, dass** der Ausdruck des Parameters $F_0$ wenigstens zum Einsatz bringt:

   - einen Parameter, bezogen auf das Wechseln zwischen einer ersten Phase an konstanter Vervielfältigungsausbeute und einer zweiten Phase an nichtkonstanter Vervielfältigungsausbeute,
   - einen Parameter, bezogen auf die konstante Vervielfältigungsausbeute bei der ersten Phase, und
   - einen Parameter, bezogen auf die nichtkonstante Vervielfältigungsausbeute bei der zweiten Phase.

7. Verfahren nach einem der vorhergehenden Ansprüche, **dadurch gekennzeichnet, dass** der Marker von Nukleinsäuren ein fluoreszierender Marker ist.

8. Verfahren nach einem der vorhergehenden Ansprüche, **dadurch gekennzeichnet, dass** die interessierende Probe eine biologische Entnahme aufweist, die möglicherweise einen Krankheitserreger enthält.

9. Verfahren nach einem der vorhergehenden Ansprüche, **dadurch gekennzeichnet, dass** die Vervielfältigungsreaktion eine Echtzeit-Polymerase-Kettenreaktion, PCR, ist.

10. Verfahren nach einem der vorhergehenden Ansprüche, **dadurch gekennzeichnet, dass** die Referenzparameter unabhängig sind von wenigstens einem der Elemente der Gruppe von experimentellen Bedingungen wie folgt: verwendetes Gerät, Typ des verwendeten Gerätes, Bedienungsperson, Periode der Gültigkeit der Reagenz-Charge, Art der Extraktion der Nukleinssäuren aus der Zielsequenz.

11. Verfahren nach einem der vorhergehenden Ansprüche, **dadurch gekennzeichnet, dass** die interessierende Probe mehrere verschiedene Zielsequenzen aufweist.

12. Gerät (AP) zum Messen der Menge an Nukleinsäuren wenigstens einer Zielsequenz in einer interessierenden Probe, mit:

   a. einem Trägerelement (U.SUP) zum Tragen wenigstens einer Probe (PROBE), die eine Reagenz-Charge und die Zielsequenz mit wenigstens einem Marker von Nukleinsäure(n) aufweist, der spezifisch ist für die Zielsequenz,
   b. einer Vervielfältigungseinheit (U: AMP), um die interessierende Probe einer Vervielfältigungsbehandlung zu unterwerfen, die aufeinander folgende Vervielfältigungszyklen, i; von 1 bis n, aufweist,
   c. einer Messeinheit (U.MES), zum Messen einer physikalischen Größe $F_i$, die repräsentativ ist für der Entwicklung des Markers für wenigstens einen Teil der Vervielfältigungszyklen,
   d. einer Verarbeitungseinheit (U.TRT), die einen Speicher aufweist und ausgerüstet ist für:

   iii. Ausdrücken eines Parameters $F_0$, basierend auf den Messungen der physikalischen Größe, der der physikalischen Größe des Markers von Nukleinsäure(n) vor jeglichem der Vervielfältigungszyklen entspricht,
   iv. Schätzen der Ausgangsmenge $N_0^{\acute{e}ch}$ an Nukleinsäure(n) einer Zielsequenz, die in der interessierenden Probe vorhanden ist, mit Hilfe einer Umwandlungsregel, basierend auf kontextabhängigen Parametern, und angewendet auf den Parameter $F_0$,

   e. einer Steuereinheit (CNTR), die in Lage ist, wenn eine interessierende Probe (PROBE) auf dem Trägerelement (U.SUP) empfangen wird, die Vervielfältigungseinheit (U.AMP) und die Messeinheit (U.MES) auf die empfangene Probe (PROBE) einwirken zu lassen, und die Verarbeitungseinheit (U.TRT) mit den erhaltenen Messungen durch die Messeinheit (U.MES) aufzurufen,

   **dadurch gekennzeichnet, dass** die kontextabhängigen Parameter Referenzparameter sind, die im Wesentlichen unabhängig von wenigstens einem Teil der experimentellen und in der Verarbeitungseinheit (U.TRT) vorgespeicherten Bedingungen sind, und vorab, ausgehend von dem Verhalten wenigstens einer Referenzprobe vom selben biologischen Format wie die interessierende Probe und von bekannter Ausgangsmenge $N_0^{\acute{r}ef}$ an Nukleinsäure(n), bestimmt wurde.

13. Computerprogrammprodukt, das Instruktionen für die Durchführung des Verfahrens nach einem der Ansprüche 1 bis 11 aufweist und dazu dient, in dem Speicher der Verarbeitungseinheit (U.TRT) in dem Gerät (AP) gemäß Anspruch 12 gespeichert zu werden.

14. Datenspeicherträger, **dadurch gekennzeichnet, dass** er Instruktionen für die Durchführung des Verfahrens nach einem der Ansprüche 1 bis 11 aufweist.

EP 2 212 820 B1

Figure 1

Figure 2

Figure 3

Figure 4

Figure 5

Figure 6

**Figure 7**

**Figure 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5210015 A
- US 5487972 A
- US 5723591 A
- US 5118801 A
- US 5925517 A
- US 6150097 A
- EP 1700245 A
- EP 11700245 A
- EP 1700145 A

**Littérature non-brevet citée dans la description**

- **R. G. Rutledge ; C. Côté.** Mathematics of quantitative kinetic PCR and the application of standard curves. *Nucleic Acids Research,* 2003, vol. 31 (16